# EUROPÄISCHE PATENTSCHRIFT

(11) **EP 2 409 253 B1**
(45) Veröffentlichungstag und Bekanntmachung des Hinweises auf die Patenterteilung: **05.12.2018**
(21) Anmeldenummer: 10707833.9
(22) Anmeldetag: 04.03.2010
(51) Int. Cl.: G06F 19/00, A61B 5/00, A61B 5/145, G16H 50/50

(54) **VERFAHREN ZUM AUTOMATISCHEN ERZEUGEN EINES NUTZERSPEZIFISCHEN MESSDATENERFASSUNGSREGIMES FÜR EINE DISKONTINUIERLICHE BUTZUCKERMESSUNG SOWIE DATENVERARBEITUNGSVORRICHTUNG UND BLUTZUCKERMESSGERÄT**
METHOD FOR AUTOMATED CREATION OF A USER-SPECIFIC MEASUREMENT DATA CREATION REGIME FOR NON-CONTINUOUS BLOOD SUGAR MEASUREMENT, DATA PROCESSING DEVICE AND BLOOD SUGAR MEASURING DEVICE
PROCÉDÉ DE PRODUCTION AUTOMATIQUE D'UN RÉGIME DE SAISIE DE DONNÉES DE MESURE SPÉCIFIQUE À L'UTILISATEUR POUR UNE MESURE DISCONTINUE DE LA GLYCÉMIE AINSI QUE DISPOSITIF DE TRAITEMENT DES DONNÉES ET LECTEUR DE GLYCÉMIE

(30) Priorität: 16.03.2009 EP 09003737
(43) Veröffentlichungstag der Anmeldung: 25.01.2012
(73) Patentinhaber: Roche Diabetes Care GmbH, 68305 Mannheim (DE); F. Hoffmann-La Roche AG, 4070 Basel (CH)
(72) Erfinder: AMANN-ZALAN, Ildiko, 69469 Weinheim (DE); TRAUTH, Bernhard, 68163 Mannheim (DE)
(74) Vertreter: Bittner, Thomas L.
(86) Internationale Anmeldenummer: PCT/EP2010/001360
(87) Internationale Veröffentlichungsnummer: WO 2010/105743

(56) Entgegenhaltungen:
- WO-A-2008/067245
- GB-A- 2 436 873
- GB-A- 2 443 434
- US-A1- 2008 269 714
- US-A1- 2009 054 753
- GROSS T M ET AL: "Performance evaluation of the MiniMed continuous glucose monitoring system during patient home use." DIABETES TECHNOLOGY & THERAPEUTICS SPRING 2000, Bd. 2, Nr. 1, April 2000 (2000-04), Seiten 49-56, XP002545136 ISSN: 1520-9156

## Beschreibung

Die Erfindung betrifft ein Verfahren zum automatischen Erzeugen eines nutzerspezifischen Messdatenerfassungsregimes für eine diskontinuierliche Blutzuckermessung mit einem Blutzuckermessgerät sowie eine Datenverarbeitungsvorrichtung und Blutzuckermessgerät.

### Hintergrund der Erfindung

Für an Diabetes, insbesondere Diabetes Mellitus erkrankte Personen ist es von besonderer Bedeutung, ihren Blutzuckerwert stets in einer bestimmten Höhe zu halten. Voraussetzung hierfür ist die Kenntnis über den Blutzuckerwert, welcher deshalb mit einem für diese Zwecke eingerichteten Blutzuckermessgerät gemessen wird. Blutzuckermessgeräte sind in verschiedenen Ausführungsformen als solche bekannt.

Wird anhand von gemessenen Werten festgestellt, dass der Blutzuckerwert einen angestrebten Bereich überschritten hat, ist ein Medikament zu verabreichen, beispielsweise mittels Spritzen von Insulin oder der oralen Verabreichung von Metformin, bei dem es sich um ein orales Antidiabetikum handelt. Wird der angestrebte Bereich für den Blutzuckerwert unterschritten, muss Zucker oral, zum Beispiel über die Nahrung oder ein Getränk, zugeführt werden. Tritt über einen längeren Zeitraum eine Überschreitung des vorgegebenen Blutzuckerbereiches auf, besteht die Gefahr von ernsthaften gesundheitlichen Komplikationen wie Blindheit, Nierenschädigung, Absterben von Gliedmaßen oder Neuropathie. Besteht die Überschreitung des vorgegeben Blutzuckerwertes kurzfristig aber erheblich, kann dies zu Übelkeit, Schwindel, Schweißausbrüchen oder sogar zu Zuständen der Verwirrtheit führen. Daher ist es von besonderer Bedeutung, dass ein Diabetiker jederzeit seinen Blutzuckerstatus kennt und gegebenenfalls eigenständig geeignete Maßnahmen einleiten kann, um ein Ausbrechen des Blutzuckerwertes aus dem angestrebten Bereich zu vermeiden.

Ein Blutzuckermessgerät, mit dem der Blutzuckerwert vom Diabetiker gemessen werden kann, ist beispielsweise aus dem Dokument DE 10 2004 057 503 A1 bekannt und wird von der Anmelderin unter der eingetragenen Marke Accu-Chek vertrieben.

Es ist bekannt, Blutzuckermessungen einem kontinuierlichen Messregime entsprechend durchzuführen. Derartige Messungen werden auch als CGM-Messungen bezeichnet. Hierbei wird über einem zusammenhängenden Zeitraum fortdauernd der Blutzuckerwert gemessen, so dass zum Beispiel der Verlauf für den Blutzuckerwert über einen gesamten Tag erfasst wird. Die Auswertung der gemessenen Blutzuckerwerte kann eine Mittelung von mehreren Tagesverläufen vorsehen. Es ist auf diese Weise möglich, tageszeitabhängige Blutzuckerwertschwankungen zu erfassen. Ein Nachteil der kontinuierlichen Blutzuckermessung besteht darin, dass einerseits hohe Kosten verursacht werden und andererseits Unannehmlichkeiten für den Diabetiker entstehen, wobei letzteres seine Ursache insbesondere im dauerhaften Tragen eines subkutanen Sensors hat. Dieses kann beim Diabetiker zu Infektionen an der Einstichstelle, Pflasterunverträglichkeit oder auch Hautirritationen führen, was ein dauerhaftes oder über einen längeren Zeitraum, zum Beispiel mehrere Monate, dauerndes Tragen durch den Diabetiker verhindert. Eine kontinuierliche Messung des Blutzuckerwertes wird zum Beispiel in dem Dokument Gross et al., "Performance Evaluation of the MiniMed Continuous Glucose Monitoring System During Patient Home Use", Diabetes Technology & Therapeutics, 2(2000)49 beschrieben.

Des Weiteren sind diskontinuierliche Blutzuckermessungen bekannt, die insbesondere auch als SMBG-Messungen (Selbst-Messungen von Blut-Glucose) bezeichnet werden und dadurch charakterisiert sind, dass Blutzuckerwerte in zeitlichen Abständen im Rahmen von Einzelmessungen und / oder einzelnen Messfolgen erfasst werden. Auf diese Weise ist es ermöglicht, mit Hilfe derartiger Blutzuckermessungen, die auch als strukturierte Blutzuckermessungen bezeichnet werden, Blutzuckerwerte in enger zeitlicher Beziehung zu bestimmten Ereignissen zu messen, zum Beispiel in Verbindung mit Mahlzeiten. Nachteile im Zusammenhang mit der strukturierten Blutzuckermessung können sich dann ergeben, wenn Ereignisse, die für den Blutzuckerwert des Diabetikers relevant sind, zwischen den Messzeitpunkten auftreten, so dass sie nicht detektierbar sind. Im Dokument US 2009/0054753 A1 ist ein Verfahren beschrieben, bei dem ausgehend von einer Einzelblutzuckermessung im Rahmen einer diskontinuierlichen Messung der Zeitpunkt für eine folgende weitere Einzelmessung in Abhängigkeit von Parametern ermittelt wird. Die Parameter berücksichtigen Umgebungs- und Patientenbedingungen.

### Zusammenfassung der Erfindung

Aufgabe der Erfindung ist es, ein Verfahren sowie eine Datenverarbeitungsvorrichtung zum automatischen Erzeugen eines nutzerspezifischen Messdatenerfassungsregimes für eine diskontinuierliche Blutzuckermessung mit einem Blutzuckermessgerät anzugeben, die eine verbesserte individuelle Blutzuckerwertkontrolle für Diabetiker ermöglichen.

Diese Aufgabe wird erfindungsgemäß durch ein Verfahren zum automatischen Erzeugen eines nutzerspezifischen Messdatenerfassungsregimes für eine diskontinuierliche Blutzuckermessung nach dem unabhängigen Anspruch 1 sowie eine Datenverarbeitungsvorrichtung nach dem unabhängigen Anspruch 9 gelöst. Weiterhin umfasst die Erfindung ein Blutzuckermessgerät nach Anspruch 10 sowie ein Computerprogramm-Produkt nach dem unabhängigen Anspruch 11. Vorteilhafte Ausgestaltungen der Erfindung sind Gegenstand von abhängigen Unteransprüchen.

Die Erfindung schafft die Voraussetzung für eine sowohl in nutzrelevanter Hinsicht als auch unter Kostengesichtspunkten optimierte Blutzuckermessung mit einem Blutzuckermessgerät. In der Datenverarbeitungsvorrichtung werden unter Verwendung von Verlaufsdaten für einen erwarteten Verlauf des Blutzuckerwertes automatisch ein Messregime für die diskontinuierliche Blutzuckermessung ermittelt und zugeordnete nutzerspezifische Messregimedaten bereitgestellt, welches aufgrund der berücksichtigen Verlaufsdaten zu einer individuell angepassten Blutzuckermessung führt.

Die bereitgestellten Verlaufsdaten geben für einen Patienten einen zu erwartenden kontinuierlichen Verlauf des Blutzuckerwertes, bevorzugt ausgehend von gemessenen Daten. Erfasst werden von den Verlaufsdaten zeitabhängige und wahlweise auch ereignisabhängige Komponenten, die den zu erwartenden Blutzuckerverlauf beeinflussen können. Letzteres hängt bei der Verlaufsbestimmung davon ab, ob ereignisbedingte Änderungen des Blutzuckerwertes auftreten, die dann auch mit erfasst werden. Gemessene Verlaufsdaten können vor dem Ableiten des Messregimes für die diskontinuierliche Blutzuckermessung verarbeitet werden, zum Beispiel im Rahmen einer Glättung der Messkurve. Auch kann vorgesehen sein, einzelne separat erfasste Blutzuckerwerte in den kontinuierlichen Kurvenverlauf einzuarbeiten, zum Beispiel um den Blutzuckerwert beeinflussende Ereignisse wie Nahrungsaufnahme, eine sportliche Aktivität oder eine Medikamentenverabreichung zu berücksichtigen. Im Ergebnis ist Ausgangspunkt der automatischen Ermittlung des Messregimes für die diskontinuierliche Blutzuckermessung ein kontinuierlicher zu erwartender Verlauf für den Blutzuckerwert.

Die Bereitstellung der Verlaufsdaten für den erwarteten Verlauf des Blutzuckerwertes des Nutzers kann derart erfolgen, dass die Verlaufsdaten ganz oder teilweise über die Dateneingangsschnittstelle zur Recheneinheit übertragen werden. Von derart bereitgestellten Verlaufsdaten können sowohl berechnete als auch experimentelle Blutzuckerwertdaten umfasst sein. Es kann aber auch ergänzend oder alternativ vorgesehen sein, dass die kontinuierlichen Verlaufsdaten in der Recheneinheit selbst ganz oder teilweise erzeugt werden. Die Verlaufsdaten zeigen einen kontinuierlichen oder durchgehenden Verlauf für wenigstens einen zusammenhängenden Zeitraum an.

Die nutzerspezifischen Messregimedaten, die insbesondere für eine so genannte SMBG-Messung bereitgestellt werden, geben Messereignisse an, die zum Beispiel mittels Angabe einer Tageszeit festgelegt sind. Ergänzend oder alternativ kann jedoch auch vorgesehen sein, dass Messereignisse in Beziehung zu einem anderen Ereignis festgelegt werden, zum Beispiel einer Medikamenteneinnahme oder einer Mahlzeit. In diesem Zusammenhang kann es vorgesehen sein, dass die nutzerspezifischen Messregimedaten einen zeitlichen Abstand zu einem solchen Ereignis angeben, nach dem die Blutzuckermessung auszuführen ist. Es ist auf diese Weise ermöglicht, unter Berücksichtigung beliebiger Verlaufsdaten, die den erwarteten Verlauf für den Blutzuckerwert des Diabetikers angeben oder Eigenschaften des Blutzuckerwertes, insbesondere anhand von zuvor experimentell gemessenen Parameterwerten anzeigen, Messregimedaten automatisch bereitzustellen, so dass für den Diabetiker nutzerspezifisch Messereignisse vorgeschlagen werden. Die Blutzuckermessung mit einem Blutzuckermessgerät kann dann von dem Diabetiker selbst oder einer anderen Person, beispielsweise einem Arzt oder einer Schwester, vorgenommen werden. Da die so erfassten Blutzuckerwerte zu individuell für den Diabetiker festgelegten Zeitpunkten oder Ereignissen gemessen werden, ergibt sich eine optimierte und individualisierte Überwachung des Blutzuckerspiegels des Diabetikers.

Es bleiben mit der Erfindung die Vorteile der diskontinuierlichen Blutzuckermessung, die auch als strukturierte Blutzuckermessung bezeichnet wird, gegenüber kontinuierlichen Blutzuckermessungen, die ihrerseits üblicher Weise für einen begrenzten zusammenhängenden Zeitraum ausgeführt werden, erhalten, beispielsweise die fehlende Notwendigkeit, des dauerhaften Tragens eines Sensors durch den Diabetiker. Auch kann der im Zusammenhang mit Blutzuckermessungen entstehende Kostenaufwand minimiert werden, da das automatisch erzeugte Messdatenerfassungsregime die Blutzuckermessungen auf notwendige Ereignisse beschränkt. Gegenüber bekannten diskontinuierlichen oder strukturierten Blutzuckermessungen ergibt sich der Vorteil einer gezielten, individualisierten Blutzuckerwertdatenerfassung, so dass der bei bekannten diskontinuierlichen Blutzuckermessungen bestehende Nachteil des Verpassens von blutzuckerwertrelevanten Ereignissen für den Diabetiker vermieden werden kann.

Während bei bekannten Verfahren entweder der zeitliche Abstand zwischen Zeitpunkten für Messungen in einem diskontinuierlichen Messregime aus bekannten Ergebnissen diskontinuierlicher Messungen abgeleitet wird oder alternativ die Messung kontinuierlich ausgeführt wird, kombiniert die Erfindung verschiedene Blutzuckermessarten, indem aus kontinuierlichen Daten für einen zu erwartenden Blutzuckerwertverlauf ein Messregime für eine diskontinuierliche Messung abgeleitet wird. Zur Ermittlung des Messregimes für die diskontinuierliche Blutzuckermessung wird hierbei nicht die ereignisgesteuerte Diskontinuität der SMBG-Messungen herangezogen, wie dies im Stand der Technik der Fall ist, sondern ein zu erwartender kontinuierlicher Verlauf. Es wurde überraschend gefunden, dass aus einem solchen Verlauf dem Messzweck entsprechend Zeitpunkte für das Messregime einer diskontinuierlichen Blutzuckermessung ableitbar sind.

Es hat sich überraschenderweise herausgestellt, dass der Blutzuckerwertverlauf patientenspezifisch dargestellt werden kann. Beispielsweise sind erhöhte Blutzuckermesswerte in Abhängigkeit vom "Biorhythmus" des Patienten zu erkennen, ohne dass eine Nahrungsaufnahme erfolgt. So sind jedoch nicht nur zu hohe oder zu niedrige Messwerte bis hin zu Hypo- oder Hyperwerten patientenspezifisch zu gewissen Tageszeiten zu erwarten. Darüber hinaus hat sich gezeigt, dass Patienten in Abhängigkeit ihres Biorhythmus Bereiche aufweisen, bei denen der Blutzuckerwert besonders instabil ist und somit starke Schwankungen aufweist. Weitere Anwendungsbeispiele beschreiben den Verlauf des Blutzuckerwertes während der Nacht, wobei Patienten häufig eine Unterzuckerung während des Schlafes zeigen. Zusammenfassend lässt sich somit sagen, dass der Blutzuckerwertverlauf patientenspezifisch von den jeweiligen physiologischen Bedingungen des Patienten mit dominiert wird und somit nicht nur ereignisabhängig beeinflusst wird.

Anders als im Stand der Technik, bei dem ausschließlich von einem ereignisabhängigen Blutzuckerwertverlauf ausgegangen wird, erlaubt das vorgeschlagene Verfahren eine "Mustererkennung" des Blutzuckerverlaufes, die für den jeweiligen Patienten spezifisch ist. Hierbei kommt es weniger auf die absoluten Blutzuckerwerte an, die zu erwarten sind, sondern vielmehr auf den relativen Verlauf der Blutzuckerwerte, der sich überraschenderweise für den Patienten als spezifisch erweist, selbst wenn unterschiedliche Ereignisse den Tagesablauf des Patienten beeinflussen. Basierend auf der Erkenntnis, dass die Mustererkennung des Blutzuckerwertverlaufes nicht nur ereignisabhängig ist, sondern patientenspezifisch beeinflusst wird, ist eine Vorhersage des Blutzuckerwertverlaufes für den Patienten auch bei verschiedenen Tagesabläufen ereignisunabhängig möglich. Insbesondere können somit personenspezifisch die Bereiche des Blutzuckerwertverlaufes ermittelt werden, in denen Schwankungen des Blutzuckerwertes erwartet werden. Ausgehend von dem relativen Blutzuckerwertverlauf der Vergangenheit kann folglich auf den relativen Blutzuckerwertverlauf in der Zukunft geschlossen werden. Vorteilhafterweise werden basierend auf der relativen Blutzuckerwertverlauf die Bereiche eines Patienten ermittelt, die als besonders kritisch bewertet werden, da hier starke Schwankungen vorkommen oder extreme Werte zu erwarten sind, sodass diese Bereiche gezielt überwacht werden können. Mit der Spotmonitoring-Methode können dann die absoluten / tatsächlichen Blutzuckerwerte zu dem jeweiligen Zeitpunkt ermittelt werden, sodass eine gezielte Kontrolle des Blutzuckerwertes für die zuvor als kritisch bewerteten Bereiche eines Patienten möglich ist. Hierfür werden in einer bevorzugten Ausführungsform Verlaufsbereiche insbesondere basierend auf der Steigung der Blutzuckerwertverlaufkurve bestimmt.

Ergänzend oder alternativ können Bereiche bestimmt werden, in denen der Blutzuckerwert des Patienten überwiegend konstant verläuft, sodass auf diese Weise Messungen eingespart werden können, zu den Zeitpunkten, bei dem für den Patienten von einem konstanten Blutzuckerwert ausgegangen werden kann. Ein Patient, der beispielsweise am Vormittag starke Schwankungen im Blutzuckerwert aufweist, jedoch am Nachmittag einen überwiegend konstanten Wert zeigt, erhält entsprechend die Empfehlungen, gezielt Messungen während des Vormittages engmaschig durchzuführen, während jedoch auf ein engmaschiges Monitoring während des Nachmittags verzichtet werden kann. Es können so das Messverhalten des Patienten optimiert und überflüssige Messungen vermieden werden. Trotz der reduzierten Anzahl der Messungen erlaubt das Verfahren dennoch eine quasi kontinuierliches Überwachen der Blutzuckerwerte, da mittels der gezielten Messregime-Daten eine Interpolation zwischen den Messdaten möglich ist und sich der Blutzuckerwertverlauf quasi kontinuierlich abbilden lässt.

Die bevorzugte Weiterbildung der Erfindung sieht vor, dass die nutzerspezifischen Messregimedaten ausgehend von ursprünglichen nutzerspezifischen Messregimedaten als optimierte nutzerspezifische Messregimedaten erzeugt werden. Bei dieser Ausgestaltung wird von bereits vorhandenen ursprünglichen nutzerspezifischen Messregimedaten ausgegangen, die mittels der Datenverarbeitungsvorrichtung unter Verwendung der bereitgestellten kontinuierlichen Verlaufsdaten optimiert werden. So kann beispielsweise vorgesehen sein, dass für einen Diabetiker bereits individualisierte und nutzerspezifische Messregimedaten existieren und nun zu Optimierungs- oder Vergleichszwecken erstmalig oder ergänzend experimentelle Daten für eine kontinuierliche Blutzuckermessung erfasst wurden, beispielsweise zum Ermitteln eines aktualisierten Tageszeitverlaufes des Blutzuckerspiegels, um schließlich hiervon ausgehend das nutzerspezifische Messregime zu bestimmen.

Bei einer zweckmäßigen Ausgestaltung der Erfindung kann vorgesehen sein, dass die kontinuierlichen Verlaufsdaten mit ermittelten Verlaufsdaten für den Blutzuckerwert des Nutzers bereitgestellt werden, die in der Recheneinheit erzeugt werden. Bei dieser Ausführungsform werden die Verlaufsdaten ganz oder teilweise in der Recheneinheit selbst erzeugt, zum Beispiel mittels Auswerten von experimentellen Blutzuckerwerten.

Die vorteilhafte Ausführungsform der Erfindung sieht vor, dass Einflussparameterdaten, welche Eigenschaften eines oder mehrerer für den Blutzuckerwert des Nutzers relevanter Einflussparameter angeben, an der Dateneingangsschnittstelle bereitgestellt werden und die ermittelten Verlaufsdaten unter Berücksichtigung der Einflussparameterdaten in der Recheneinheit erzeugt werden. Die Einflussparameter können genutzt werden, um den erwarteten kontinuierlichen Verlauf des Blutzuckerwertes ganz oder teilweise zu ermitteln; sie können selbst mittels Datenverarbeitung berechnet oder experimenteller Natur sein. Auch können sie in einer Ausgestaltung herangezogen werden, um einen schon existierenden Verlauf des Blutzuckerwertes zu prüfen oder zu ändern. Die Einflussparameterdaten, die insbesondere zuvor gemessene Messdaten für den Blutzuckerwert des Diabetikers aus einer oder mehreren Messungen umfassen, können mit Hilfe eines elektronischen Speichermediums zur Verfügung gestellt werden, welches mit der Datenverarbeitungsvorrichtung beispielsweise über eine drahtlose oder eine drahtgebundene Datenkommunikationsverbindung verbunden ist. Auch kann in einer Ausgestaltung vorgesehen sein, dass die Datenverarbeitungsvorrichtung an ein Netzwerk gekoppelt ist.

In einer anderen Ausgestaltung kann ein nutzerspezifisches Messregime erstellt werden, um die Messereignisse für den Diabetiker für ein neues Medikament oder eine geänderte Medikamentenverabreichung anzupassen. In diesem Fall betreffen die Einflussparameterdaten elektronische Informationen über das Medikament und / oder seine Verabreichung, soweit hierdurch ein Einfluss auf den Blutzuckerspiegel des Diabetikers erwartet wird. Unabhängig davon, welche Art von Einflussparameterdaten für das Erzeugen der optimierten nutzerspezifischen Messregimedaten herangezogen werden, besteht mit dieser Ausführungsform des Verfahrens die Möglichkeit, auf aktuelle Änderungen zu reagieren, wenn notwendig auch mehrfach.

Es ist vorgesehen, dass die Einflussparameterdaten gemessene Blutzucker-Messdaten aus einer kontinuierlichen Blutzuckermessung umfassend bereitgestellt werden. Bei den Blutzucker-Messdaten aus der kontinuierlichen Blutzuckermessung handelt es sich um individuell ermittelte Daten, die über einen längeren Zeitraum im Rahmen einer kontinuierlichen Messung erfasst wurden, zum Beispiel über mehrere Tage oder Wochen. Solche experimentellen Daten geben dann selbst bereits einen endgültigen oder vorläufigen erwarteten kontinuierlichen Verlauf für den Blutzuckerwert oder Anhaltspunkte hierfür an.

Eine vorteilhafte Ausführungsform der Erfindung sieht vor, dass die Einflussparameterdaten Eigenschaften eines oder mehrerer Einflussparameter ausgewählt aus der folgenden Gruppe von Einflussparametern angebend bereitgestellt werden: Tageszeitabhängigkeit des Glucosewertes, Nahrungsmittelart, Nahrungsmittelaufnahme, Medikamentenart, Medikamenteneinnahme, personenspezifische Einflussparameter wie Alter, Gewicht, Bodymaßindex, eine physische Aktivität, Stress, eine Comorbidität und / oder eine akute Erkrankung. Allgemein können die Einflussparameterdaten in elektronischer Form einzelne der vorgenannten Einflussparameter oder eine beliebige Kombination hiervon betreffen. Auf diese Weise ist eine weitere Individualisierung der nutzerspezifischen Messregimedaten ermöglicht, da individuell für den Diabetiker eine gewünschte Kombination von Einflussparameter berücksichtigt werden kann.

Bei einer zweckmäßigen Fortbildung der Erfindung kann vorgesehen sein, dass die kontinuierlichen Verlaufsdaten mit gemessenen Verlaufsdaten in Form von Blutzucker-Messdaten aus einer Blutzuckermessung für den Nutzer bereitgestellt werden, die über die Dateneingangsschnittstelle empfangen werden. Die gemessenen Verlaufsdaten können zum Beispiel als Blutzucker-Messdaten aus einer kontinuierlichen Blutzuckermessung bereitgestellt werden. Es handelt es sich hierbei um experimentell ermittelte Daten, die über einen längeren Zeitraum im Rahmen einer kontinuierlichen Messung erfasst wurden, zum Beispiel über mehrere Tage oder Wochen. In diesem Zusammenhang kann es vorgesehen sein, die gemessenen Blutzucker-Messdaten aus mehreren kontinuierlichen Blutzuckermessungen zu mitteln. Auch können die kontinuierlichen Blutzucker-Messdaten im Zusammenhang mit einer Medikamentenverabreichung stehen, um so den Einfluss einer Medikamenteneinnahme zu erfassen und bei der automatischen Erzeugung des nutzerspezifischen Messdatenerfassungsregimes zu berücksichtigen. Als Messparameter werden in diesem Zusammenhang beispielsweise die postprandiale Glucoseexkursion und die Zeit bis zum Erreichen einer maximalen Glucosekonzentration, was auch als "*time to peak*" bezeichnet wird, herangezogen. Die Glucoseexkursion betrifft die Höhe des postprandialen Glucoseanstiegs nach einer Mahlzeit oder einer Nahrungsaufnahme. Die vorgenannten Parameter, die beispielsweise auch im Rahmen von Studien ausgewertet werden, können aus dem zeitlichen Verlauf der kontinuierlichen Blutzuckermessung bestimmt werden. Diese Werte selbst oder hieraus abgeleitete Einflussparameter können dann für das Erzeugen der nutzerspezifischen Messregimedaten herangezogen werden, insbesondere auch zur Optimierung von ursprünglichen nutzerspezifischen Messregimedaten.

Bevorzugt sieht eine Fortbildung der Erfindung vor, dass die nutzerspezifischen Messregimedaten in dem Blutzuckermessgerät erzeugt oder über die Datenausgangsschnittstelle auf das Blutzuckermessgerät übertragen werden. Das Erzeugen der nutzerspezifischen Messregimedaten in dem Blutzuckermessgerät selbst bedeutet, dass die Datenverarbeitungsvorrichtung in dieses integriert ist, so dass eine "vor Ort" Erzeugung der Messregimedaten erfolgt. Alternativ kann vorgesehen sein, dass die Datenverarbeitungsvorrichtung getrennt von dem Blutzuckermessgerät gebildet ist, so dass die nutzerspezifischen Messregimedaten an das Blutzuckermessgerät übertragen werden, entweder über eine drahtlose oder eine drahtgebundene Datenkommunikationsverbindung. Unabhängig davon, ob die nutzerspezifischen Messregimedaten in dem Blutzuckergerät oder in einer hiervon getrennten Datenverarbeitungsvorrichtung bereitgestellt werden, kann das Bereitstellen und / oder das Ausgeben der nutzerspezifischen Messregimedaten über die Datenausgabeeinrichtung mit einer optischen und / oder einer akustischen Signalisierung verbunden werden, wodurch die Aufmerksamkeit des Nutzers gezielt geweckt wird. Beim Vorliegen der nutzerspezifischen Messregimedaten in dem Blutzuckermessgerät kann vorgesehen sein, dass über eine Datenausgabeeinrichtung des Blutzuckermessgerätes, zum Beispiel eine Anzeige, eine Signalisierung für den Benutzer erfolgt, so dass dieser an anstehende Messereignisse, die sich aus dem Messdatenerfassungsregime ergeben, einmal oder mehrfach erinnert wird. Dieses kann mit einer optischen und / oder einer akustischen Signalausgabe gekoppelt sein. Auf diese Weise wird für den Benutzer die Handhabung des Blutzuckermessgerätes verbessert, da der Diabetiker automatisch und dem nutzerspezifischen Messregime entsprechend über anstehende Messdatenerfassungen informiert wird.

Bei einer vorteilhaften Ausgestaltung der Erfindung kann vorgesehen sein, dass das Verfahren weiterhin die folgenden Schritte umfasst: Erzeugen von Steuerdaten für eine Messbetriebsart des Blutzuckermessgerätes unter Verwendung der nutzerspezifischen Messregimedaten in dem Blutzuckermessgerät und Steuern der Messbetriebsart des Blutzuckermessgerätes entsprechend den Steuerdaten. Das Steuern der Messbetriebsart des Blutzuckermessgerätes entsprechend den Steuerdaten wird mit einer von dem Blutzuckermessgerät umfassten Steuereinrichtung ausgeführt, bei der es sich beispielsweise um eine Prozessor-Speicher-Einheit handelt. Das automatische Steuern in der Messbetriebsart kann beispielsweise darin bestehen, dass den nutzerspezifischen Messregimedaten entsprechend eine Information für den Nutzer ausgegeben wird, so dass dieser an den Messzeitpunkt im Rahmen der diskontinuierlichen Blutzuckermessung erinnert wird.

Eine Weiterbildung der Erfindung kann vorsehen, dass mittels des Blutzuckermessgerätes Blutzuckermesswerte für den Nutzer erfasst und den nutzerspezifischen Messregimedaten und / oder den Steuerdaten zugeordnet werden. Dieses kann so ausgeführt werden, dass gemessene Blutzuckerwerte den in den nutzerspezifischen Messregimedaten definierten Messereignissen zugeordnet werden. Auf diese Weise kann es beispielsweise ermöglicht sein, gemessene Blutzuckerwerte dem nutzerspezifischen Messdatenregime entsprechend auszuwerten.

Eine bevorzugte Weiterbildung der Erfindung sieht vor, dass die nutzerspezifischen Messregimedaten einem zeitlichen Messregime entsprechend zeitversetzt und den nutzerspezifischen Messereignissen entsprechend mittels einer Datenausgabeeinrichtung ausgegeben werden. Alternativ oder ergänzend zur zeitversetzten Ausgabe eines Teils der nutzerspezifischen Messregimedaten kann vorgesehen sein, einen teilweisen oder vollständigen Ablaufplan als Übersicht für die Messereignisse des nutzerspezifischen Messregimes auszugeben. In einer Ausführungsform kann vorgesehen sein, den teilweisen oder vollständigen Ablaufplan gemäß dem nutzerspezifischen Messregime dem Diabetiker zu übermitteln, zum Beispiel über ein drahtloses Kommunikationsnetz auf ein mobiles Gerät wie ein Handy oder einen Laptop. Auch kann die Bereitstellung der nutzerspezifischen Messregimedaten über das Internet oder ein anderes elektronisches Netzwerk vorgesehen sein.

Bei einer zweckmäßigen Ausgestaltung der Erfindung kann vorgesehen sein, dass die nutzerspezifischen Messregimedaten mit Wichtungsinformation erzeugt werden, welche den nutzerspezifischen Messereignissen des Messregimes für die diskontinuierliche Blutzucker-Messdatenerfassung eine Wichtung zuordnet. Die Wichtung, die auch als Priorisierung der nutzerspezifischen Messereignisse bezeichnet werden kann, ermöglicht eine noch weitergehende Individualisierung des nutzerspezifischen Blutzuckermessregimes. Zunächst können dem nutzerspezifischen Messregime entsprechend erfasste Blutzuckerwerte bei der Auswertung der Wichtung gemäß berücksichtigt werden, so dass zum Beispiel einzelne Blutzuckerwerte eine höhere Wichtung als andere erhalten. Auch kann ergänzend oder alternativ hierzu in einer Ausgestaltung vorgesehen sein, die Signalisierung für den Nutzer der Wichtung entsprechend zu gestalten. Beispielsweise kann die Anzeige für ein nutzerspezifisches Messereignis am Blutzuckermessgerät bei unterschiedlicher Wichtung in verschiedener Art und Weise erfolgen, zum Beispiel mittels Ausgabe eines farblich angepassten Signals, beispielsweise eine rote Signalisierung höchst Priorität anzeigt. Auch kann vorgesehen sein, den Benutzer mittels Signalisierung am Blutzuckermessgerät auf höher gewichtete Messereignisse mehrfach und auf weniger gewichtete Messereignisse nur einmal hinzuweisen.

Die vorteilhafte Weiterbildung der Erfindung kann vorsehen, dass beim Erzeugen von nutzerspezifischen Messregimedaten die kontinuierlichen Verlaufsdaten analysiert werden, um Verlaufsbereiche zu ermitteln, für die wenigstens eine Änderung des Blutzuckerwertes des Nutzers erwartet wird, und die nutzerspezifischen Messregimedaten unter Berücksichtigung der ermittelten Verlaufsbereiche erzeugt werden. Die Berücksichtigung der ermittelten Verlaufsbereiche, in denen Änderungen des Blutzuckerwertes erwartet werden, bedeutet zum Beispiel, dass für den ermittelten Verlaufsbereich zugeordnete Zeitabschnitte oder in zeitlicher Relation hierzu das Messregime eine oder mehrere Messungen vorschlägt.

Ergänzend kann bei einer Ausführung der Erfindung vorgesehen sein, beim Erzeugen der nutzerspezifischen Messregimedaten die kontinuierlichen Verlaufsdaten analysiert werden, um weitere Verlaufsbereiche zu ermitteln, für die eine im Wesentlichen konstanter Verlauf des Blutzuckerwertes erwartet wird, um hieraus messfreie Zeitabschnitte für die diskontinuierliche Blutzuckermessung abzuleiten.

In Verbindung mit der Datenverarbeitungsvorrichtung zum Erzeugen eines nutzerspezifischen Messdatenerfassungsregimes für eine diskontinuierliche oder strukturierte Blutzuckermessung können die folgenden vorteilhaften Ausgestaltungen vorgesehen sein. Eine zweckmäßige Fortbildung der Erfindung sieht vor, dass die Recheneinheit konfiguriert ist, die nutzerspezifischen Messregimedaten ausgehend von ursprünglichen nutzerspezifischen Messregimedaten als optimierte nutzerspezifische Messregimedaten zu erzeugen. Bevorzugt sieht eine Weiterbildung der Erfindung vor, dass die Recheneinheit konfiguriert ist, die nutzerspezifischen Messregimedaten mit Wichtungsinformation zu erzeugen, welche den nutzerspezifischen Messereignissen des Messregimes für die diskontinuierliche Blutzucker-Messdatenerfassung eine Wichtung zuordnet. Weiterhin kann vorgesehen sein, dass die Recheneinheit konfiguriert ist, die Verlaufsdaten mit ermittelten Verlaufsdaten für den Blutzuckerwert des Nutzers bereitzustellen. In einer Ausführung der Erfindung ist vorgesehen, dass die Dateneingangsschnittstelle konfiguriert ist, Einflussparameterdaten, welche Eigenschaften eines oder mehrerer für den Blutzuckerwert des Nutzers relevanter Einflussparameter angeben, zu empfangen, und die Recheneinheit konfiguriert ist, die ermittelten Verlaufsdaten unter Berücksichtigung der Einflussparameterdaten zu erzeugen.

Die Datenverarbeitungsvorrichtung kann in einer Ausführung in ein Blutzuckermessgerät integriert sein. Bei dieser Ausgestaltung kann vorgesehen sein, dass die Recheneinheit konfiguriert ist, Steuerdaten für eine Messbetriebsart unter Verwendung der nutzerspezifischen Messregimedaten zu erzeugen. Bei einer weiteren Ausführung kann vorgesehen sein, dass die Recheneinheit konfiguriert ist, für den Nutzer erfasste Blutzuckermesswerte den nutzerspezifischen Messregimedaten und / oder den Steuerdaten zuzuordnen.

### Beschreibung bevorzugter Ausführungsbeispiele der Erfindung

Die Erfindung wird im Folgenden anhand von Bevorzugten Ausführungsbeispielen unter Bezugnahme auf Figuren einer Zeichnung näher erläutert. Hierbei zeigen:
- Fig. 1: eine schematische Blockdarstellung einer Datenverarbeitungsvorrichtung zum automatischen Erzeugen eines nutzerspezifischen Messdatenerfassungsregimes für eine diskontinuierliche Blutzuckermessung mit einem Blutzuckermessgerät,
- Fig. 2: eine graphische Darstellung des gemessenen zeitlichen Verlaufs des Blutzuckerwertes eines Patienten in einer kontinuierliche Blutzuckermessung für einen ersten Messtag und
- Fig. 3: eine graphische Darstellung des gemessenen zeitlichen Verlaufs des Blutzuckerwertes eines Patienten in einer kontinuierliche Blutzuckermessung für einen zweiten Messtag.

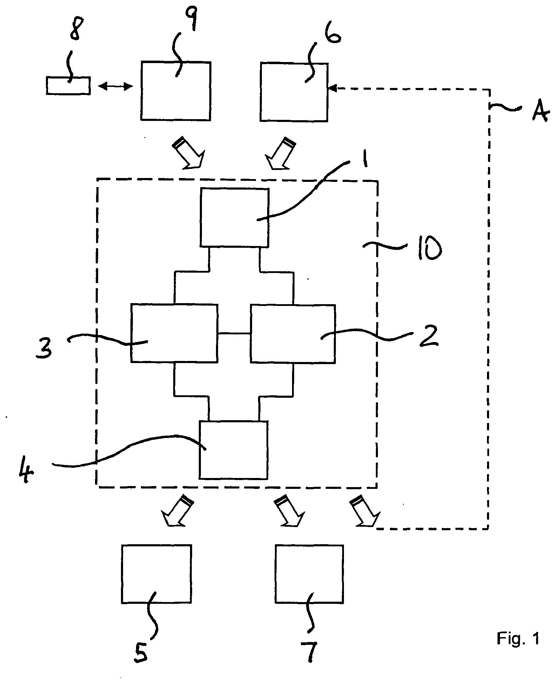

Fig. 1 zeigt eine schematische Darstellung einer Datenverarbeitungsvorrichtung 10 zum automatischen Erzeugen eines nutzerspezifischen Messdatenerfassungsregimes für eine diskontinuierliche oder strukturierte Blutzuckermessung mit einem Blutzuckermessgerät. An eine Dateneingangsschnittstelle 1 sind eine Speichereinrichtung 2 sowie eine Recheineinheit 3, die mit der Speichereinrichtung 2 verbunden ist, angeschlossen. Die Speichereinrichtung 2 und die Recheneinheit 3 stehen weiterhin mit einer Datenausgangsschnittstelle 4 in Verbindung, über die elektronische Daten ausgegeben werden können, bei dem Ausführungsbeispiel in Fig. 1 an eine Datenausgabeeinrichtung 5 und / oder ein Blutzuckermessgerät 6, wobei letzteres in Fig. 1 mittels einer gestrichelten Linie A schematisch gezeigt ist. Bei der Datenausgabeeinrichtung 5, die alternativ zur Ausführung in Fig. 1 in die Datenverarbeitungsvorrichtung 10 integriert sein kann, handelt es sich zum Beispiel um ein Display. Auch eine alternative oder ergänzende Sprachausgabe kann vorgesehen sein. Ermöglicht ist mit der Datenausgangsschnittstelle 4 auch eine Datenübertragung auf ein Speichermedium 7, zum Beispiel ein tragbares Speichermedium.

Im Betrieb der Datenverarbeitungsvorrichtung 10 werden an der Dateneingangsschnittstelle 1 Einflussparameterdaten bereitgestellt, welche Eigenschaften eines oder mehrerer für den Blutzuckerwert eines Nutzers relevanter Einflussparameter anzeigen. Hierbei kann es sich bereits um Daten handeln, die einen erwarteten Verlauf des Blutzuckerwertes für den Diabetiker vollständig oder teilweise anzeigen, weshalb diese Daten dann zumindest als Teil der Verlaufsdaten herangezogen werden. Es kann aber auch vorgesehen sein, dass die Einflussparameterdaten einen beliebigen Parameter und dessen Wirkung oder Einfluss auf den Blutzuckerwert angeben. Diese Einflussparameterdaten werden dann in der Recheneinheit 3 verarbeitet, um einen erwarteten Verlauf für den Blutzuckerwert zu erzeugen oder einen bereits existierenden Verlauf den Einflussparametern entsprechend abzuändern.

Bei der in Fig. 1 dargestellten Ausführungsform werden Einflussparameterdaten direkt vom Blutzuckermessgerät 6 übermittelt, mit dem zuvor eine kontinuierliche Blutzuckermessung für den Nutzer durchgeführt wurde. Zur Datenübertragung kann hierbei eine drahtlose oder eine drahtgebundene Datenkommunikationsverbindung genutzt werden. Auch eine Bereitstellung mittels eines Speichermittels 8, welches dann zum Beispiel mit einem an die Dateneingangsschnittstelle 1 angeschlossenen Lesegerät 9 ausgelesen wird, kann vorgesehen sein. Auf diesem Wege können im Übrigen auch Daten von der Datenverarbeitungsvorrichtung 10 ausgegeben werden, wobei das Lesegerät 9 in diesem Fall als Schreib-Lesegerät ausgeführt sein kann oder ein separates Schreibgerät vorgesehen ist.

Die über die Dateneingangsschnittstelle 1 empfangenen Daten werden dann wahlweise in der Speichereinrichtung 2 zwischengespeichert und mit der Recheneinheit 3 verarbeitet, um nutzerspezifische Messregimedaten zu erzeugen, die ein nutzerspezifisches Messregime für eine diskontinuierliche Blutzucker-Messdatenerfassung angeben. Die Datenverarbeitung in der Recheneinheit 3 umfasst das Bestimmen des nutzerspezifischen Messregimes. Sie kann das Bestimmen des zu erwartenden Verlaufs des Blutzuckerwertes als Vorstufe umfassen, sofern die Verlaufsdaten nicht schon bereitgestellt sind. Die Auswertung der kontinuierlichen Verlaufsdaten umfasst das Bestimmen von Verlaufsbereichen, in denen eine Änderung des Blutzuckerwertes erwartet wird, um hierfür dann eine oder mehrere Messungen vorzuschlagen.

Die erzeugten nutzerspezifischen Messregimedaten werden anschließend von der Recheneinheit 3 an die Datenausgangsschnittstelle 4 übergeben, um sie dort bereitzustellen und zum Beispiel an die Datenausgabeeinrichtung 5 zu übermitteln. Alternativ oder ergänzend ist vorgesehen, die nutzerspezifischen Messregimedaten an das Blutzuckermessgerät 6 zu übermitteln, so dass die nutzerspezifischen Messregimedaten im Blutzuckermessgerät 6 vorliegen. Dort können die nutzerspezifischen Messregimedaten dann genutzt werden, um beispielsweise den Nutzer über anstehende Messereignisse gemäß dem nutzerspezifischen Messregime zu informieren.

Die Datenausgabeeinrichtung 5 kann über ein Netzwerk, in welches die Datenverarbeitungsvorrichtung integriert ist, mit dieser verbunden sein, zum Beispiel über ein drahtloses Kommunikationsnetzwerk oder ein drahtgebundenes Kommunikationsnetzwerk, beispielsweise das Internet. Die netzwerkbasierte Ausgestaltung hat den Vorteil, dass beispielsweise in einer zentralen Datenverarbeitungsvorrichtung für eine Vielzahl von Diabetikern nutzerspezifische Messregimedaten erzeugt werden können, die dann individuell über das Netzwerk verteilt werden. Einem Diabetiker können auf diese Art und Weise auf einem von ihm genutzten Gerät, bei dem es sich nicht notwendigerweise um sein Blutzuckermessgerät 6 handelt, die nutzerspezifischen Messregimedaten zur Verfügung gestellt werden, zum Beispiel auf einem Mobilfunktelefon oder einem Computer. Falls gewünscht, kann der Nutzer die nutzerspezifischen Messregimedaten dann von seinem Gerät auf das Blutzuckermessgerät 6 übertragen, zum Beispiel mit Hilfe eines transportablen elektronischen Speichermediums.

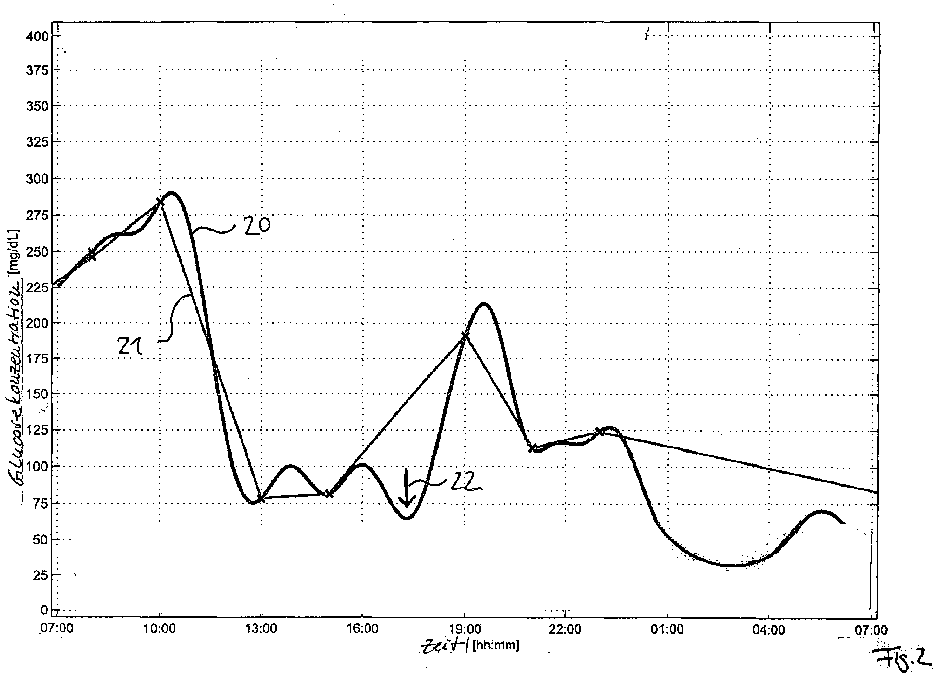

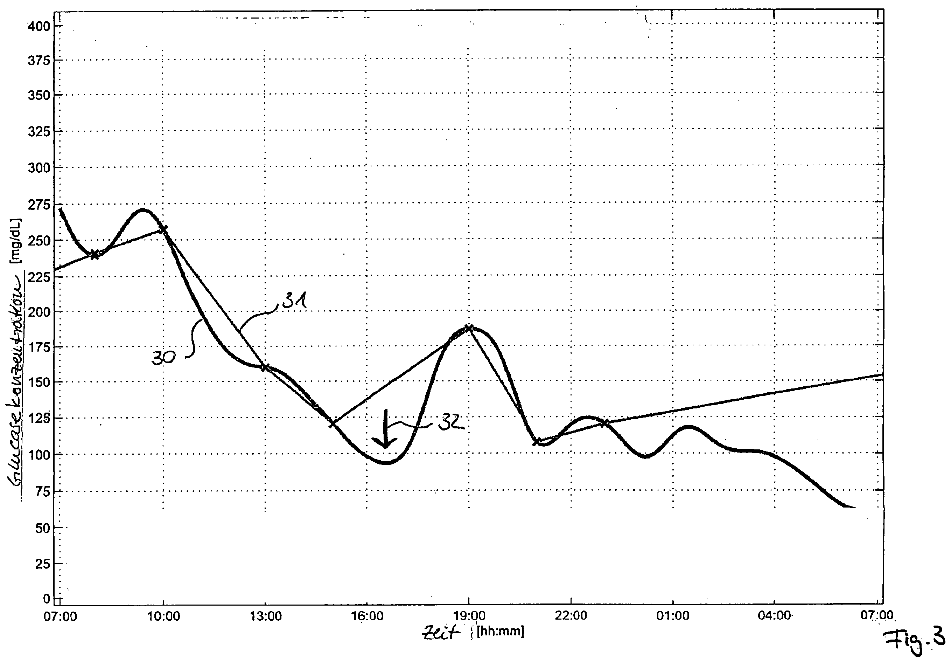

Fig. 2 und 3 zeigen eine graphische Darstellung für den Blutzuckerverlauf für einen Patienten, der im Rahmen einer kontinuierlichen Blutzuckermessung experimentell bestimmt wurde. Es ist der gemessene zeitliche Verlauf des Blutzuckerwerts für einen ersten und einen zweiten Messtag dargestellt.

Die Darstellung in den Fig. 2 und 3 zeigen jeweils mittels einer ersten Kurve 20, 30 den gemessenen Verlauf der kontinuierlichen Blutzuckermessung für 24 Stunden, also Verlaufsdaten für den Blutzuckerwert. Entlang dieser Kurve sind dann mit Hilfe von Kreuzen Messzeitpunkte für eine diskontinuierliche Blutzuckermessung, insbesondere eine SBMG-Messung, dargestellt, die mittels einer jeweiligen zweiten Kurve 21, 31 verbunden sind. Es ergibt sich, dass in beiden grafischen Darstellungen in den Fig. 2 und 3 jeweils um etwa 17 Uhr der Verlauf der kontinuierlichen Blutzuckermessung ein charakteristisches Kurvental durchläuft.

Wenn nun den Kreuzen in den Fig. 2 und 3 entsprechend eine diskontinuierliche Blutzuckermessung durchgeführt wird, geht die Information über diesen charakteristischen "Ausreißer" des Blutzuckerspiegels des Diabetikers verloren. Die automatische Auswertung der gemessenen Blutzuckerwerte mit Hilfe der vorangehend beschriebenen Datenverarbeitungsvorrichtung 10 führt nun dazu, dass nutzerspezifische Messregimedaten erzeugt werden, die ergänzend oder alternativ eine Blutzuckermessung im Rahmen der diskontinuierlichen Messung für etwa 17:30 Uhr vorschlagen, da für diesen Zeitbereich eine Änderung des Blutzuckerwertes im Verlauf ermittelt wurde. Dieses ist in den Fig. 2 und 3 mittels Pfeilen 22, 32 schematisch dargestellt. Auf diese Weise wird das ursprünglich gewählte Messregime für die diskontinuierliche Messung nutzerspezifisch optimiert. Konkret wird die 7-Punkt-Messung um einen Messpunkt um 17:30 Uhr ergänzt. Die Messregimedaten werden dann ausgehend von Fig. 2 zum Beispiel die in Tabelle 1 dargestellten Informationen umfassend bereitgestellt, wobei der in Tabelle 1 unterstrichene Messzeitpunkt "17:30Uhr" Ausfluss der Berücksichtigung der Messwerte für die kontinuierliche Blutzuckermessung ist.

**Tabelle 1**

| Lfd. Nummer | Messzeit |
|---|---|
| 1 | 8:00Uhr |
| 2 | 10:00Uhr |
| 3 | 13:00Uhr |
| 4 | 15:00Uhr |
| 5 | 17:30Uhr |
| 6 | 19:00Uhr |
| 7 | 21:00Uhr |
| 8 | 23 :00Uhr |

## Patentansprüche

1. Verfahren zum automatischen Erzeugen eines nutzerspezifischen Messdatenerfassungsregimes für eine diskontinuierliche Blutzuckermessung mit einem Blutzuckermessgerät (6) in einer Datenverarbeitungsvorrichtung, die eine Dateneingangsschnittstelle (1), eine Speichereinrichtung (2), eine Recheneinheit (3) und eine Datenausgangsschnittstelle (4) aufweist, wobei das Verfahren die folgenden Schritte umfasst:
- Bereitstellen von Einflussparameterdaten, welche gemessene Blutzucker-Messdaten aus einer kontinuierlichen Blutzuckermessung umfassen, an der Dateneingangsschnittstelle (1),
- Erzeugen von Verlaufsdaten, welche einen erwarteten kontinuierlichen Verlauf für den Blutzuckerwert eines Nutzers angeben, mittels der Recheneinheit (3), aus den Einflussparameterdaten,
- Analysieren, mittels der Recheneinheit (3), der kontinuierlichen Verlaufsdaten mittels Mustererkennung,
- Bestimmen, mittels der Recheneinheit (3), von Verlaufsbereichen in den Verlaufsdaten, für die wenigstens eine Änderung des Blutzuckerwerts des Nutzers erwartet wird,
- Bestimmen, mittels der Recheneinheit (3), von Messzeitpunkten, zu denen eine Blutzuckermessung ausgeführt werden soll, anhand der Verlaufsbereiche,
- Erzeugen von nutzerspezifischen Messregimedaten, die ein Messregime mit nutzerspezifischen Messereignissen für eine diskontinuierliche Blutzuckermessung mit einem Blutzuckermessgerät angeben, unter Berücksichtigung der Verlaufsdaten mittels der Recheneinheit (3), wobei die Messereignisse durch die Messzeitpunkte bestimmt sind, und
- Bereitstellen der nutzerspezifischen Messregimedaten an der Datenausgangsschnittstelle (4) für eine Datenübertragung.

2. Verfahren nach Anspruch 1, **dadurch gekennzeichnet, dass** die Einflussparameterdaten Eigenschaften eines oder mehrerer Einflussparameter ausgewählt aus der folgenden Gruppe von Einflussparametern umfassen:
- Tageszeitabhängigkeit des Blutzuckerwertes,
- Nahrungsmittelart,
- Nahrungsmittelaufnahme,
- Medikamentenart,
- Medikamenteneinnahme,
- personenspezifische Einflussparameter wie Alter, Gewicht, Bodymaßindex, eine physische Aktivität, Stress, eine Comorbidität und / oder eine akute Erkrankung.

3. Verfahren nach mindestens einem der vorangehenden Ansprüche, **dadurch gekennzeichnet, dass** die Datenverarbeitungsvorrichtung in das Blutzuckermessgerät (6) integriert ist und die nutzerspezifischen Messregimedaten in dem Blutzuckermessgerät (6) erzeugt werden, oder dass die Datenverarbeitungsvorrichtung von dem Blutzuckermessgerät (6) getrennt gebildet ist und die nutzerspezifischen Messregimedaten über die Datenausgangsschnittstelle (4) auf das Blutzuckermessgerät (6) übertragen werden.

4. Verfahren nach Anspruch 3, **dadurch gekennzeichnet, dass** das Verfahren weiterhin die folgenden Schritte umfasst:
- Erzeugen von Steuerdaten für eine Messbetriebsart des Blutzuckermessgerätes (6) unter Verwendung der nutzerspezifischen Messregimedaten in dem Blutzuckermessgerät (6) und
- Steuern der Messbetriebsart des Blutzuckermessgerätes (6) entsprechend den Steuerdaten.

5. Verfahren nach Anspruch 3 oder 4, **dadurch gekennzeichnet, dass** mittels des Blutzuckermessgerätes (6) Blutzuckermesswerte für den Nutzer erfasst und den nutzerspezifischen Messregimedaten und / oder den Steuerdaten zugeordnet werden.

6. Verfahren nach mindestens einem der vorangehenden Ansprüche, **dadurch gekennzeichnet, dass** die nutzerspezifischen Messregimedaten einem zeitlichen Messregime entsprechend zeitversetzt und den nutzerspezifischen Messereignissen entsprechend mittels einer Datenausgabeeinrichtung (5) ausgegeben werden.

7. Verfahren nach mindestens einem der vorangehenden Ansprüche, **dadurch gekennzeichnet, dass** die nutzerspezifischen Messregimedaten mit Wichtungsinformation erzeugt werden, welche den nutzerspezifischen Messereignissen des Messregimes für die diskontinuierliche Blutzuckermessung eine Wichtung zuordnet.

8. Verfahren nach mindestens einem der vorangehenden Ansprüche, **dadurch gekennzeichnet, dass** beim Erzeugen der nutzerspezifischen Messregimedaten die Verlaufsdaten analysiert werden, um weitere Verlaufsbereiche zu ermitteln, für die ein im Wesentlichen konstanter Verlauf des Blutzuckerwertes erwartet wird, um hieraus messfreie Zeitabschnitte für die diskontinuierliche Blutzuckermessung abzuleiten.

9. Datenverarbeitungsvorrichtung zum Erzeugen eines nutzerspezifischen Messdatenerfassungsregimes für eine diskontinuierliche Blutzuckermessung mit einem Blutzuckermessgerät, insbesondere gemäß einem Verfahren nach mindestens einem der vorangehenden Ansprüche, mit:
- einer Dateneingangsschnittstelle (1), die konfiguriert ist, Einflussparameterdaten, welche gemessene Blutzucker-Messdaten aus einer kontinuierlichen Blutzuckermessung umfassen, zu empfangen,
- einer Speichereinrichtung (2),
- einer Recheneinheit (3), die konfiguriert ist,
- Verlaufsdaten, welche einen erwarteten kontinuierlichen Verlauf für den Blutzuckerwert eines Nutzers angeben, aus den Einflussparameterdaten zu erzeugen,
- die kontinuierlichen Verlaufsdaten mittels Mustererkennung zu analysieren,
- Verlaufsbereiche in den Verlaufsdaten zu bestimmen, für die wenigstens eine Änderung des Blutzuckerwerts des Nutzers erwartet wird,
- anhand der Verlaufsbereiche Messzeitpunkte zu bestimmen, zu denen eine Blutzuckermessung ausgeführt werden soll,
- unter Berücksichtigung der Verlaufsdaten nutzerspezifische Messregimedaten zu erzeugen, die ein Messregime mit nutzerspezifischen Messereignissen für eine diskontinuierliche Blutzuckermessung mit einem Blutzuckermessgerät (6) angeben, wobei die Messereignisse durch die Messzeitpunkte bestimmt sind und
- einer Datenausgangsschnittstelle (4), die konfiguriert ist, die nutzerspezifischen Messregimedaten für eine Datenübertragung bereitzustellen.

10. Blutzuckermessgerät mit einer Datenverarbeitungsvorrichtung nach Anspruch 9.

11. Computerprogramm-Produkt mit Programmcode, welcher wahlweise auf einem computerlesbaren Speichermedium gespeichert ist und welcher geeignet ist, beim Ablauf auf einer Rechenvorrichtung ein Verfahren nach mindestens einem der Ansprüche 1 bis 8 auszuführen.

## Claims

1. Method of automatically generating a user-specific measuring data recording routine for discontinuous blood glucose measurement with a blood glucose measuring device (6) in a dataprocessing device, which has a data input interface (1), a storage device (2), a calculation unit (3) and a data output interface (4), whereby the method comprises the following steps:
- providing influencing parameter data which comprise measured blood glucose data from a continuous blood glucose measurement at the data input interface (1),
- generating process data indicating an expected continuous course of the blood glucose value of a user, by the calculation unit (3), from the influencing parameter data,
- analysing, by the calculation unit (3), the continuous process data by means of pattern recognition,
- determining, by the calculation unit (3), periods in the process data in which at least one change to the user's blood glucose level is expected,
- determining, by the calculation unit (3), of measurement times, at which a blood glucose measurement shall be conducted, by the periods,
- generating of user-specific measuring routine data, which indicate a measuring routine with user-specific measuring events for a discontinuous blood glucose measurement with a blood glucose meter taking into account the process data by means of the calculation unit (3), wherein the measuring events are determined by the measurement times, and
- providing of the user-specific measuring routine data to the data output interface (4) for data transmission.

2. Method according to claim 1, **characterised in that** the influencing parameter data comprise properties of one or more influencing parameters selected from the following group of influencing parameters:
- Dependency of the blood glucose value on the time of day,
- Type of food,
- Food intake,
- Type of medication,
- Medication use,
- Person-specific influencing parameters such as age, weight, body mass index, physical activity, stress, a comorbidity and/or or an acute illness.

3. Method according to at least one of the preceding claims, **characterised in that** the dataprocessing device is integrated into the blood glucose measurement device (6) and the user-specific measuring routine data are generated in the blood glucose measuring device (6), or **in that** the dataprocessing device is separated from the blood glucose measurement device (6) and the user-specific measuring routine data are transferred to the blood glucose measuring device (6) via the data output interface (4).

4. Method according to claim 3, **characterised in that** the method also comprises the following steps:
- generating of control data for a measuring mode of the blood glucose measuring device (6) using the user-specific measuring routine data in the blood glucose measuring device (6) and
- controlling the measuring mode of the blood glucose measuring device (6) in accordance with the control data.

5. Method in accordance with claim 3 or 4, **characterised in that** by means of the blood glucose measuring device (6) blood glucose values for the user are recorded and assigned to the user-specific measuring routine data and/or the control data.

6. Method according to at least one of the preceding claims, **characterised in that** the user-specific measuring routine data are outputted by means of a data outputting device (5) time-delayed in accordance with a temporal measuring routine and in accordance with the user-specific measuring events.

7. Method according to at least one of the preceding claims, **characterised in that** user-specific measuring routine data are generated with weighting information which assigns a weighting to the user-specific measuring events of the measuring routine for discontinuous blood glucose measurement.

8. Method according to at least one of the preceding claims **characterised in that** during the generation of the user-specific measuring routine data the process data are analysed in order to determine further periods for which an essentially constant course of the blood sugar value is expected, in order to derive measurement-free periods for the discontinuous blood glucose measurement.

9. Dataprocessing device for generating a user-specific measuring data routine for discontinuous blood glucose measurement with a blood glucose measuring device, more particularly in accordance with a method according to at least one of the preceding claims, with:
- a data input interface (1) which is configured to receive influencing parameter data, which comprise measured blood glucose data from a continuous blood glucose measurement,
- a storage device (2),
- a calculation unit (3) which is configured for
- generating process data indicating an expected continuous course of the blood glucose value of a user, from the influencing parameter data,
- analysing the continuous process data by means of pattern recognition,
- determining periods in the process data in which at least one change to the user's blood glucose level is expected,
- determining of measurement times, at which a blood glucose measurement shall be conducted, by the periods,
- taking into account the process data, generating user-specific measuring routine data indicating a measuring routine with user-specific measuring events for a discontinuous blood glucose measurement with a blood glucose measuring device (6), whereby the measuring events are determined from the measurement times, and
- a data output interface (4) which is configured for providing the user-specific measuring routine data for data transmission.

10. Blood glucose measuring device comprising a data processing device according to claim 9.

11. Computer program product comprising a program code, which is optionally stored on a computer-readable storage medium and which is suitable when running on a calculation device of executing a method according to at least one of claims 1 to 8.

## Revendications

1. Procédé, destiné à la génération automatique d'un régime de détection de données mesurées spécifique à un utilisateur pour une mesure discontinue de la glycémie à l'aide d'un glucomètre (6) dans un dispositif de traitement de données qui comprend une interface d'entrée de données (1), un système de mémoire (2), un module de calcul (3) et une interface de sortie de données (4), le procédé comportant les étapes suivantes, consistant à :
- mettre à disposition des données de paramètres d'influence, lesquelles comprennent des données de mesure de la glycémie mesurées, provenant d'une mesure continue de la glycémie sur l'interface d'entrée de données (1),
- générer des données d'historique, lesquelles indiquent une courbe continue attendue de la valeur de glycémie d'un utilisateur, à l'aide du module de calcul (3), à partir des données de paramètres d'influence,
- analyser, à l'aide du module de calcul (3), les données d'historique continues, par identification de modèle,
- déterminer, à l'aide du module de calcul (3), des zones de courbes dans les données d'historique, pour lesquelles est attendue au moins une modification de la valeur de glycémie de l'utilisateur,
- déterminer, à l'aide du module de calcul (3) des moments de mesure auxquels une mesure de la glycémie doit être réalisée, à l'aide des zones de courbe,
- générer des données de régime de mesure, qui indiquent un régime de mesure comportant des événements de mesure spécifiques à l'utilisateur pour une mesure discontinue de la glycémie à l'aide d'un glucomètre, sous considération des données d'historique au moyen du module de calcul (3), les événements de mesure étant déterminés par les moments de mesure et
- mettre à disposition des données de régime de mesure spécifiques à l'utilisateur sur l'interface de sortie de données (4), aux fins d'un transfert de données.

2. Procédé selon la revendication 1, **caractérisé en ce que** les données de paramètres d'influence comprennent des propriétés d'un ou de plusieurs paramètres d'influence, choisis dans le groupe suivant de paramètres d'influence :
- la dépendance de l'heure dans la journée de la valeur de glycémie,
- le type d'alimentation,
- l'absorption des aliments,
- les types de médicaments,
- la prise de médicaments,
- les paramètres d'influence spécifiques à la personne, tels que l'âge, le poids, l'indice de masse corporelle, une activité physique, le stress, une comorbidité et/ou une pathologie aiguë.

3. Procédé selon au moins l'une quelconque des revendications précédentes, **caractérisé en ce que** le dispositif de traitement de données est intégré dans le glucomètre (6) et les données de régime de mesure spécifiques à l'utilisateur sont générées dans le glucomètre (6) ou **en ce que** le dispositif de traitement de données est conçu en étant séparé du glucomètre (6) et les données de régime de mesure spécifiques à l'utilisateur sont transférées via l'interface de sortie de données (4) sur le glucomètre (6).

4. Procédé selon la revendication 3, **caractérisé en ce que** le procédé comprend par ailleurs les étapes suivantes, consistant à :
- générer des données de commande pour un mode de mesure du glucomètre (6), en utilisant les données de régime de mesure spécifiques à l'utilisateur dans le glucomètre (6) et
- commander le mode de mesure du glucomètre (6), en fonction des données de commande.

5. Procédé selon la revendication 3 ou 4, **caractérisé en ce qu'**à l'aide du glucomètre (6), des valeurs de mesure de la glycémie sont détectées pour l'utilisateur et associées aux données de régime de mesure spécifiques à l'utilisateur et/ou aux données de commande.

6. Procédé selon au moins l'une quelconque des revendications précédentes, **caractérisé en ce qu'**en fonction d'un régime de mesure temporel, les données de régime de mesure spécifiques à l'utilisateur sont éditées en différé et en fonction des événements de mesure spécifiques à l'utilisateur, sont éditées sur un système d'édition de données (5).

7. Procédé selon au moins l'une quelconque des revendications précédentes, **caractérisé en ce que** les données de régime de mesure spécifiques à l'utilisateur sont générées avec une information de pondération, laquelle associe aux événements de mesure spécifiques à l'utilisateur du régime de mesure une pondération pour la mesure discontinue de la glycémie.

8. Procédé selon au moins l'une quelconque des revendications précédentes, **caractérisé en ce que** lors de la génération des données de régime de mesure spécifiques à l'utilisateur, les données d'historique sont analysées pour déterminer des zones de courbe supplémentaire, pour lesquelles est attendue une courbe sensiblement constante de la valeur de glycémie, pour en déduire des périodes exemptes de mesures, pour la mesure discontinue de la glycémie.

9. Dispositif de traitement de données, destiné à la génération d'un régime de détection de données mesurées spécifique à l'utilisateur, pour une mesure discontinue de la glycémie à l'aide d'un glucomètre, notamment d'après un procédé selon au moins l'une quelconque des revendications précédentes, comportant :
- une interface d'entrée de données (1) qui est configurée pour recevoir des données de paramètres d'influence, lesquelles comprennent des données de mesure de la glycémie mesurées à partir d'une mesure continue de la glycémie,
- un système de mémoire (2),
- un module de calcul (3), qui est configuré pour -
- générer des données d'historique, lesquelles indiquent une courbe continue attendue de la valeur de glycémie d'un utilisateur, à partir des données de paramètres d'influence, qui analysent des données d'historique à l'aide d'une identification de modèle,
- déterminer des zones de courbe dans les données d'historique pour lesquelles au moins une modification de la valeur de glycémie de l'utilisateur est attendue,
- à l'aide des zones de courbe, déterminer des moments de mesure auxquels une mesure de la glycémie doit être réalisée,
- sous considération des données d'historique, générer des données de régime de mesure spécifiques à l'utilisateur, qui indiquent un régime de mesure avec des événements de mesure spécifiques à l'utilisateur pour une mesure discontinue de la glycémie à l'aide d'un glucomètre (6), les événements de mesure étant déterminés par les moments de mesure et
- une interface de sortie de données (4), qui est configurée pour mettre à disposition les données de régime de mesure spécifiques à l'utilisateur, pour un transfert de données.

10. Glucomètre comportant un dispositif de traitement de données selon la revendication 9.

11. Produit de programme informatique comportant un code programme, lequel est mémorisé au choix sur un support de mémorisation lisible par ordinateur et lequel est apte, lors de son exécution sur un dispositif informatique à réaliser un procédé selon au moins l'une quelconque des revendications 1 à 8.
